# EUROPÄISCHE PATENTANMELDUNG

(11) **EP 0 717 970 A1**
(43) Veröffentlichungstag der Anmeldung: **26.06.1996**
(21) Anmeldenummer: 95118831.7
(22) Anmeldetag: 30.11.1995
(51) Int. Cl.: A61F 9/00, A61M 1/00

(54) **Opthalmologische Aspirations- und Irrigationseinrichtung sowie Verfahren zum Betreiben derselben**

(30) Priorität: 20.12.1994 CH 3832/94
(71) Anmelder: GRIESHABER & CO. AG SCHAFFHAUSEN, CH-8203 Schaffhausen (CH)
(72) Erfinder: Grieshaber, hans, Ch-8200 Schaffhausen (CH); Vogel, Urs, CH-8200 Schaffhausen (CH)
(74) Vertreter: Althoff, Gerhard

(57) **Zusammenfassung**

Es wird eine ophthalmologische Aspirations- und Irrigationseinrichtung (100) für eine Vorrichtung (150) zur Durchführung mikrochirurgischer Eingriffe am Auge eines Lebewesens sowie ein Verfahren zum Betreiben derselben vorgeschlagen.

Die Aspirations- und Irrigationseinrichtung (100) umfasst im wesentlichen eine Infusionseinheit (10), eine Druckeinheit (30), eine mit dem Auge (40) in Verbindung stehende Irrigationseinheit (45) sowie eine ebenfalls mit dem Auge (40) in Verbindung stehende Aspirationseinheit (75). Die Aspirationseinheit (75) hat eine erste Pumpeneinheit sowie eine zweite Pumpeneinheit, wobei die erste Pumpeneinheit mindestens mit einer ersten Vakuumpumpe und die zweite Pumpeneinheit mindestens mit einer zweiten Vakuumpumpe versehen ist.

Zur Erreichung eines möglichst grossen Vakuumwertes in der ersten Pumpeneinheit wird mittels der zweiten Pumpeneinheit in einer ersten Phase, d.h. bei gleichzeitiger Aspiration des dem Auge (40) zugeführten flüssigen oder gasförmigen Mediums, ein im System der ersten Pumpeneinheit vorhandenes Luftpolster entfernt.

## Beschreibung

Die Erfindung bezieht sich auf eine ophthalmologische Aspirations- und Irrigationseinrichtung für eine Vorrichtung zur Durchführung mikrochirurgischer Eingriffe am Auge eines Lebewesens sowie auf ein Verfahren zum Betreiben derselben, wobei die Aspirations- und Irrigationseinrichtung eine Infusionseinheit, eine Druckeinheit, eine Irrigationseinheit sowie eine mit mindestens einem ersten Filter und mit mindestens einer Vakuumpumpe versehene Aspirationseinheit umfasst, mittels welcher ein dem Auge über eine damit in Verbindung stehende Irrigationsleitung zugeführtes flüssiges oder gasförmiges Medium sowie beim operativen Eingriff anfallende Gewebeteilchen oder dergleichen über mindestens eine mit dem Auge in Verbindung stehende Aspirationsleitung absaugbar sind.

In Verbindung mit einer Vorrichtung gemäss der EP-A 0 601 313 zur Durchführung mikrochirurgischer Eingriffe am Auge eines Lebewesens ist aus der EP-A 0 596 314 eine ophthalmologische Aspirations- und Irrigationseinrichtung bekannt, mittels welcher während des operativen Eingriffs unter Beibehaltung einer ausreichenden Saugleistung eine Regulierung des intraokularen Druckes (IOP) sowie bei etwaigen Zwischenfällen in Form von Blutungen oder dergleichen ein kurzzeitiges Erhöhen des intraokularen Druckes mit gasförmigem oder flüssigem Medium durchführbar ist. Bei der bekannten Aspirations- und Irrigationseinrichtung besteht die Möglichkeit, dass während des Eingriffs in der Aspirationsleitung ein unerwünschtes Luftpolster aufgebaut wird, durch welches die Vakuumpumpe durch eine zeitliche Verzögerung in ihrer Wirkung beeinträchtigt werden kann.

Der Erfindung liegt die Aufgabe zugrunde, für eine ophthalmologische Aspirations- und Irrigationseinrichtung gemäss der im Oberbegriff des Patentanspruchs 1 genannten Gattung weitere Verbesserungen aufzuzeigen sowie ein Verfahren anzugeben, mittels welchem beim Absaugen (Vakuumieren) unter Beibehaltung der erforderlichen Sterilität möglichst rasch ein ausreichend grosser Vakuumwert erreicht wird.

Die erfindungsgemässe ophthalmologische Aspirations- und Irrigationseinrichtung ist dadurch gekennzeichnet, dass eine mit dem Auge in Verbindung stehende und mindestens die erste Vakuumpumpe, den ersten Filter sowie erste Hauptleitungen umfassende erste Pumpeneinheit sowie eine über zweite Hauptleitungen damit in Verbindung stehende zweite Pumpeneinheit vorgesehen sind, wobei die zweite Pumpeneinheit mindestens eine zweite Vakuumpumpe aufweist, die der ersten Vakuumpumpe nachgeschaltet ist.

Das erfindungsgemässe Verfahren ist dadurch gekennzeichnet, dass in einer ersten Phase und bei gleichzeitiger Aspiration der Flüssigkeit aus dem Auge ein im System der ersten Pumpeneinheit vorhandenes Luftpolster von der angeschlossenen zweiten Pumpeneinheit abgesaugt und entfernt, mindestens aber teilweise abgesaugt und entfernt wird.

Weitere Merkmale der Erfindung ergeben sich aus der folgenden Beschreibung in Verbindung mit der Zeichnung und den einzelnen Patentansprüchen.

Die Erfindung wird nachstehend anhand der Zeichnung beschrieben. Es zeigt:
**Fig.1** eine in perspektivischer Ansicht dargestellte ophthalmologische Vorrichtung zur Durchführung mikrochirurgischer Eingriffe sowie eine damit in Wirkverbindung stehende Irrigations- und Aspirationseinrichtung;
**Fig.2** die als Fliessschema dargestellte Irrigations- und Aspirationseinrichtung für die ophthalmologische Vorrichtung gemäss Fig.1, und
**Fig.3** die als Blockschema dargestellte Irrigations- und Aspirationseinrichtung gemäss Fig.2 mit den wesentlichen Funktionselementen.

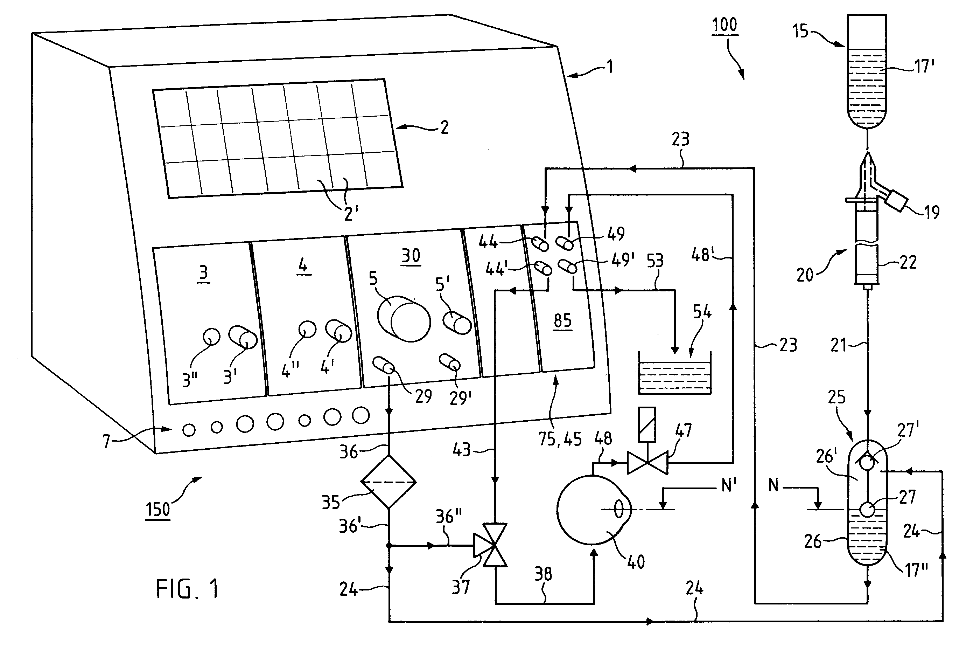

Zur Verdeutlichung der vorliegenden Erfindung ist in Fig.1 in schematischer sowie in perspektivischer Ansicht eine Vorrichtung 150 dargestellt, welche zur Durchführung mikrochirurgischer Eingriffe am Auge 40 eines Lebewesens ausgebildet ist und mit einer zugeordneten und in der Gesamtheit mit 100 bezeichneten Irrigations- und Aspirationseinrichtung in Wirkverbindung steht.

Die Vorrichtung 150 umfasst mehrere in einem Gehäuse 1 angeordneten und für den jeweiligen operativen Eingriff ausgebildete Funktionseinheiten 3, 4, 30, 45 und 75. Die einzelnen Funktionseinheiten 3, 4, 30, 45 und 75 sind beispielsweise als nicht näher dargestellte Einschübe ausgebildet, welche auswechselbar von der nicht bezeichneten Frontseite in das Gehäuse 1 einschiebbar sowie mit nicht dargestellten Mitteln im Gehäuse 1 arretierbar sind. Die beiden ersten Funktionseinheiten 3, 4 sind mit nicht dargestellten Mitteln versehen, mittels welcher der Innenraum des Auges 40 während des operativen Eingriffs beleuchtet werden kann. Hierzu wird ein nicht dargestelltes Lichtleiterkabel an entsprechend vorgesehene Buchsen 3'' und 4'' angeschlossen. Die Lichtstärke kann jeweils über einen Drehknopf 3' und 4' stufenlos reguliert werden. Unterhalb der Funktionseinheiten 3 und 4 ist zudem eine Steckerleiste 7 angeordnet, welche zum Anschluss zusätzlicher Operationsinstrumente (nicht dargestellt) vorgesehen ist.

Weiterhin ist an der Frontseite des Gehäuses 1 ein Bildfeld 2 angeordnet, welches in einzelne Anzeige- und Bedienungsfelder 2' mit LCD-Anzeige (Liquid Cristal Display) unterteilt ist. Das einzelne Anzeige- und Bedienungsfeld 2' dient während des operativen Eingriffs als programmabhängig gesteuerter Informationsaustausch für den Chirurgen. Die Anzeige- und Bedienungsfelder 2' sind für eine operationsabhängige Programmwahl durch geringfügigen Fingerdruck betätigbar. Die angewählte Anzeige oder angewählten Anzeigen sind dabei jeweils beleuchtet und dienen dem Chirurgen jeweils zur visuellen Anzeige entsprechender Messgrössen (Messwerte).

In der folgenden Beschreibung werden die Funktionseinheit 30 als Druckeinheit 30, die Funktionseinheit 45 als Irrigationseinheit 45 und die Funktionseinheit 75 als Aspirationseinheit 75 bezeichnet. Die Druckeinheit 30 hat einen Stutzen 29 zum Anschliessen einer Leitung 36 sowie einen Einstellknopf 5 zum Regulieren, vorzugsweise zum stufenlosen Regulieren der beim operativen Eingriff zur Aufrechterhaltung des intraokularen Druckes (IOP) erforderlichen Druckluftzufuhr. In dem Gehäuse 1 kann weiterhin eine nicht dargestellte Visko-Injektionseinrichtung angeordnet werden, welche über einen Einstellknopf 5' regulierbar ist und über einen Stutzen 29' mit einer nicht dargestellten Leitung in Verbindung steht.

Die einzelnen Funktionselemente der Irrigationseinheit 45 sowie der Aspirationseinheit 75 stehen über einen als Schlauchträger in Form einer Kassette ausgebildeten Einschub 85 miteinander in Wirkverbindung. Der an der Stirnseite der Druckeinheit 30 angeordnete Stutzen 29 sowie weitere an der Stirnseite des Einschubes 85 im Abstand zueinander angeordnete Stutzen 49,49' und 44,44' stehen über entsprechend angeschlossene Versorgungs- und Entsorgungsleitungen mit weiteren Funktionselementen 15, 20, 25, 35, 37, 47 und 54 der in Fig.1 teilweise dargestellten Irrigations- und Aspirationseinrichtung 100 in Wirkverbindung.

Die mit der ophthalmologischen Vorrichtung 150 gemäss Fig.1 in Wirkverbindung stehende Irrigations- und Aspirationseinrichtung 100 wird nachstehend im einzelnen beschrieben:

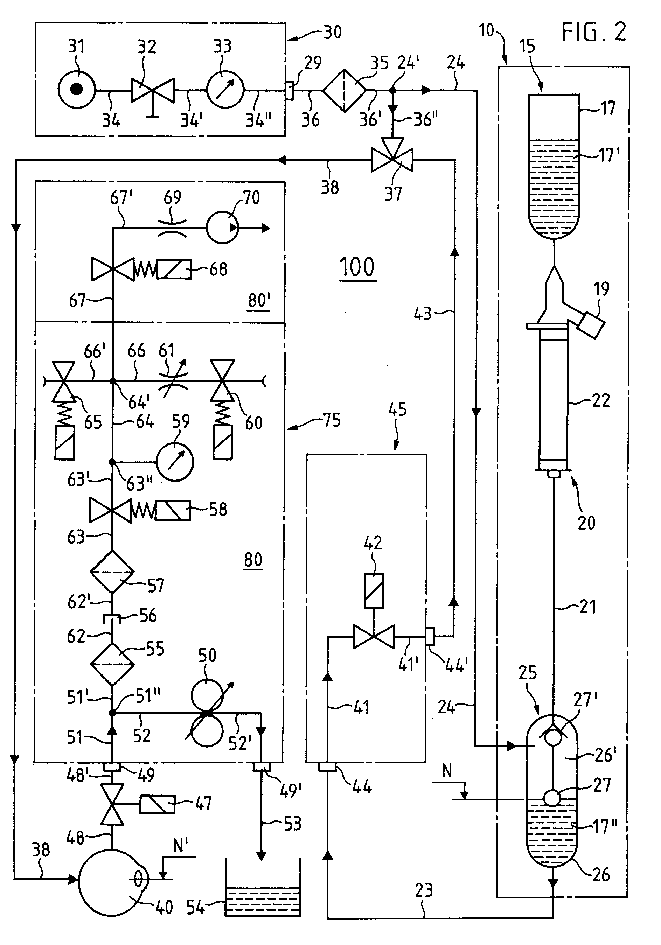

Die in Fig.2 als Fliessschema dargestellte Irrigations- und Aspirationseinrichtung 100 umfasst im dargestellten Ausführungsbeispiel die Funktionseinheiten 10; 30; 45 und 75, welche über entspechend angeschlossene Irrigations- und Aspirationsleitungen miteinander verbunden sind. Nachstehend wird die erste Einheit 10 als Infusionseinheit 10, die zweite Einheit 30 als Druckeinheit 30, die dritte Einheit 45 als Irrigationseinheit 45 und die vierte Einheit 75 als Aspirationseinheit 75 bezeichnet.

Die Infusionseinheit 10 umfasst ein erstes Infusionsgerät 15, ein zweites Infusionsgerät 25 sowie eine Tropfvorrichtung 20. Das erste Infusionsgerät 15 hat einen mit einer Salzlösung 17' gefüllten ersten Behälter 17, welcher unter Zwischenschaltung der mit einem Auffangbehälter 22 und einem daran angeordneten Filter 19 versehenen Tropfvorrichtung 20 über eine Leitung 21 mit einem zweiten Behälter 26 des zweiten Infusionsgerätes 25 verbunden ist. Im Innenraum 26' des zweiten Behälters 26 ist ein Schwimmerventil 27,27' angeordnet, mittels welchem das Niveau N der vom ersten Behälter 17 über den Auffangbehälter 22 dem zweiten Behälter 26 zugeführten Salzlösung 17'' überwacht wird. Das Niveau N der Salzlösung 17'' im Innenraum 26' des zweiten Behälters 26 kann über das mit nicht dargestellten Regelelementen in Wirkverbindung stehende Schwimmerventil 27;27' durch Zuführung der Salzlösung 17' aus dem ersten Behälter 17 konstant gehalten werden. Zur Erreichung eines vom Operateur zu bestimmenden intraokularen Druckes (IOP) wird das erste Niveau N der Salzlösung 17'' im zweiten Behälter 26 auf das lageabhängige zweite Niveau N' des in Fig.2 versetzt dazu dargestellten Auges 40 eingestellt und reguliert.

Die Druckeinheit 30 hat eine Druckquelle 31, ein Regelventil 32 sowie ein Druckmessgerät 33, wobei die einzelnen Teile 31, 32 und 33 über eine erste und zweite Leitung 34 und 34' miteinander sowie über eine dritte Leitung 34'' mit dem Anschlussstutzen 29 verbunden sind. Von dem Druckmessgerät 33 wird der mit nicht dargestellten Mitteln am Regelventil 32 eingestellte Druck erfasst und der analoge Wert für eine visuelle Überprüfung durch den Operateur an dem am Gehäuse 1 (Fig.1) angeordneten Bilfeld 2 (LCD-Anzeige) angezeigt. Der erforderliche Druck kann vor dem operativen Eingriff oder während des operativen Eingriffs von dem Chirurgen den Erfordernissen entsprechend stufenlos eingestellt und reguliert werden.

Die Irrigationseinheit 45 umfasst ein ansteuerbares Unterbrecherorgan 42, welches eingangsseitig über eine erste Leitung 41 mit dem ersten Anschlussstutzen 44 und ausgangsseitig über eine zweite Leitung 41' mit dem zweiten Anschlussstutzen 44' verbunden ist. Das Unterbrecherorgan 42 ist beispielsweise ein elektrisch ansteuerbares Magnetventil 42, welches mit einem nicht dargestellten Quetschelement versehen ist. Die beiden Leitungen 41 und 41' sind aus elastischem Material, beispielsweise aus transparentem, elastischem Kunststoff wie Silikon oder dergleichen hergestellt. Das Quetschelement wird nach Ansteuerung des Magnetventils 42 entsprechend betätigt, wodurch der Durchfluss des Mediums durch die beiden Leitungen 41 und 41' entweder unterbrochen oder freigegeben wird.

Die Aspirationseinheit 75 umfasst im dargestellten Ausführungsbeispiel eine erste und eine zweite Pumpeneinheit 80 und 80', wobei die jeweils mit mehreren Funktionselementen und Leitungen versehenen Pumpeneinheiten 80 und 80' über Hauptleitungen 64 und 67 miteinander in Verbindung stehen und nachstehend im einzelnen beschrieben werden.

Die als Ausführungsbeispiel dargestellte erste Pumpeneinheit 80 umfasst einen ersten Filter 55, ein Kupplungsstück 56, einen zweiten Filter 57, ein erstes Ventil 58, ein Druckmessgerät 59 (Sensor) sowie ein zweites Ventil 60 und drittes Ventil 65. Die vorstehenden Funktionselemente 55,56,57,58, 59,60 und 65 stehen über erste Hauptleitungen 51,51';62,62'; 63,63';64 und 66,66' und Verbindungsstellen 63'' und 64' miteinander in Verbindung. An die eine mit dem ersten Anschlussstutzen 49 in Verbindung stehende Leitung 51 ist an einer weiteren Verbindungsstelle 51'' eine Leitung 52 angeschlossen, welche unter Zwischenschaltung einer ersten Vakuumpumpe 50 als Leitungsteil 52' mit dem zweiten Anschlussstutzen 49' verbunden ist. Der erste Filter 55, die Anschlussstutzen 49,49' sowie 44,44' und die einzelnen Verbindungsleitungen 51,51';52,52'; 62 und 41,41' sind vorzugsweise auf einem als Kassette ausgebildeten Schlauchträger angeordnet. Ein bevorzugtes Ausführungsbeispiel des Schlauchträgers ist aus der eingangs erwähnten EP-A 0 601 313 bekannt.

Die erste Pumpeneinheit 80 steht über eine an dem ersten Anschlussstutzen 49 angeschlossene Leitung 48,48', vorzugsweise unter Zwischenschaltung eines ansteuerbaren Unterbrecherorgans 47, mit einem nicht dargestellten und zum Zerkleinern und Entfernen des Linsenkerns ausgebildeten augenchirurschen Instrument in Wirkverbindung. Das Unterbrecherorgan 47 ist vorzugsweise als ein mit nicht dargestellten Mitteln ansteuerbares Magnetventil 47 ausgebildet und mit einem nicht dargestellten Quetschelement versehen, mittels welchem die vorzugsweise aus transparentem, elastischem Kunststoff oder dergleichen hergestellte Leitung 48 und 48' für den Absaugvorgang (Aspiration) unterbrochen und wieder freigegeben werden kann. Ein Instruments zum Zerkleinern und Entfernen des Linsenkerns ist beispielsweise aus der EP-A 0 623 328 bekannt.

Die erste Pumpeneinheit 80 steht weiterhin über eine an dem zweiten Stutzen 49' angeschlossene Leitung 53 mit einem zugeordneten Behälter 54 in Verbindung. Mittels der ersten, peristaltisch arbeitenden Vakuumpumpe 50 wird das aus dem Auge abgesaugte Medium zusammen mit den beim operativen Eingriff anfallenden Gewebeteilchen abgesaugt und über die Leitungen 52',53 dem Behälter 54 zugeführt. Die erste, peristaltisch arbeitende Vakuumpumpe 50 ist beispielsweise mit nicht dargestellten Regulierelementen versehen, mittels welcher der in Form von Luft und Flüssigkeit anzusaugende Volumenstrom vorzugsweise variabel einstellbar ist.

An dieser Stelle wird darauf hingewiesen, dass die erste, peristaltisch arbeitende Vakuumpumpe 50 beispielsweise vor- und rückwärtslaufend ausgebildet ist. Eine bevorzugte Ausführungsform der in zusammengebautem Zustand mit dem kassettenförmigen Einschub 85 in Wirkverbindung stehenden ersten Vakuumpumpe 50 ist aus der eingangs erwähnten EP-A 0 601 313 an sich bekannt. Der nachgeschaltete erste Filter 55 dient zur Abtrennung des flüssigen Mediums von den ebenfalls nachgeschalteten Funktionselementen sowie zur Aufrechterhaltung der Sterilität derselben. Der zweite Filter 57 dient zum Ausscheiden einer den ersten Filter 55 gegebenenfalls durchdringenden Restflüssigkeit, so dass ein Eindringen von Feuchtigkeit in die nachgeschalteten Funktionselemente der Pumpeneinheiten 80,80' ausgeschlossen ist.

Das für die Belüftung der ersten Pumpeneinheit 80 vorgesehene zweite Ventil 60 steht über die Leitung 66 und Verbindungsstelle 64' mit der ersten Hauptleitung 64 in Verbindung. Dem zweiten Ventil 60 ist vorzugsweise ein Drosselorgan 61, beispielsweise ein mit nicht dargestellten Mitteln einstellbar ausgebildetes Drosselorgan 61 vorgeschaltet, mittels welchem die erste Pumpeneinheit 80 gedrosselt belüftet werden kann.

Für eine beschleunigte Belüftung (automatischer Reflux) der ersten Pumpeneinheit 80 ist an die Hauptleitung 64 die mit dem dritten Ventil 65 in Verbindung stehende Leitung 66' angeschlossen. Mit dem dritten Ventil 65 wird eine vollständige Belüftung des Aspirationssystems erreicht, sobald das Vakuum in dem System zusammenfällt. Das Zusammenfallen des Vakuums kann dann erfolgen, wenn das nicht dargestellte augenchirurgische Instrument beispielsweise durch zusammenhängende Gewebeteilchen oder dergleichen verstopft und dadurch bei laufender Vakuumpumpe 50 der Vakuumwert auf den maximalen Setwert ansteigen kann. Beim Auflösen der Verstopfung, welches beispielsweise durch plötzliches Auflösen der zusammenhängenden Gewebeteilchen sowie durch Absaugen derselben erfolgt, kann das Vakuum zusammenbrechen. Das Zusammenbrechen des Vakuums wird bei dieser Einrichtung vorzugsweise durch geeignete elektronische Mittel (nicht dargestellt) erkannt, so dass gleichzeitig eine vollständige Belüftung durch das entsprechend aktivierte dritte Ventil 65 durchgeführt werden kann.

Die zweite Pumpeneinheit 80' umfasst ausgehend von der ersten Pumpeneinheit 80 in Strömungsrichtung gesehen die zweite Hauptleitung 67 und 67', ein Ventil 68, ein Drosselorgan 69 sowie eine zweite Vakuumpumpe 70 (Booster-Pumpe). Die zweite Pumpeneinheit 80' dient im wesentlichen zum Absaugen von Luft aus der ersten Pumpeneinheit 80. Die einzelnen Funktionselemente 68,69 und 70 der zweiten Pumpeneinheit 80' stehen über die zweite Hauptleitung 67,67' miteinander in Verbindung, wobei das eine Leitungteil 67 mittels der Verbindungsstelle 64' an die erste Hauptleitung 64 der ersten Pumpeneinheit 80 angeschlossen ist. Das mit der zweiten Hauptleitung 67 verbundene sowie dem Drosselorgan 69 vorgeschaltete Ventil 68 wird jeweils bei Aktivierung der zweiten Vakuumpumpe 70 mit nicht dargestellten Mitteln geöffnet. Das Ventil 68 in der zweiten Pumpeneinheit 80' kann bei eingeschalteter und aktivierter Vakuumpumpe 70 mit nicht dargestellten Mitteln zeitverzögert geöffnet werden. Durch Ausschaltung der zweiten Vakuumpumpe 70 wird automatisch das Ventil 68 geschlossen.

Die vorstehend beschriebenen Funktionseinheiten 10; 30; 45 und 75 sind über entsprechend angeschlossene Infusions- und Aspirationsleitungen miteinander verbunden. Die Infusionseinheit 10 steht einerseits über eine erste Infusionsleitung 23 mit der Irrigationseinheit 45 und andererseits über eine zweite Infusionsleitung 24 und durch eine Verbindungsstelle 24' mit einer dritten Infusionsleitung 36,36' in Verbindung. Die dritte Infusionsleitung 36,36' ist unter Zwischenschaltung eines Filters 35 an dem einen Stutzen 29 der Druckeinheit 30 angeschlossen. An der Verbindungsstelle 24' ist ein weiteres Teilstück 36'' der dritten Infusionsleitung 36,36' sowie ein Mehrwegeventil 37 angeschlossen. Das Mehrwegeventil 37 steht einerseits über eine vierte Infusionsleitung 38 mit dem Auge 40 in Verbindung und ist andererseits über eine angeschlossene fünfte Infusionsleitung 43 an dem zweiten Stutzen 44' der Irrigationseinheit 45 angeschlossen. Die mit dem einen Ende mit der dritten Infusionsleitung 36,36' verbundene zweite Infusionsleitung 24 ist mit dem anderen Ende derart am zweiten Behälter 26 angeschlossen, dass der Behälterraum 26' oberhalb der Salzlösung 17'' von der Druckeinheit 30 über den zwischengeschalteten Filter 35 mit keimfreier Druckluft beaufschlagbar ist. Von dem zweiten Behälter 26 wird die mit Druckluft beaufschlagte Salzlösung 17'' über die am ersten Stutzen 44 der Irrigationseinheit 45 angeschlossene Leitung 23 sowie über die an dem zweiten Stutzen 44' der Irrigationseinheit 45 angeschlossene erste Infusionsleitung 43 über das Mehrwegeventil 37 sowie über die vierte Infusionsleitung 38 dem Auge 40 zugeführt.

Mittels der einzelnen, die Funktionseinheiten 10; 30 und 45 miteinander verbindenden Infusionsleitungen 23; 24; 36,36', 36''; 38 und 43 kann in einer ersten Stellung des Mehrwegeventils 37 über die Leitungen 36,36',36'' und 38 dem Auge 40 entweder keimfreie Druckluft oder aber in einer zweiten Stellung des Mehrwegeventils 37 über die Infusionsleitungen 23, 43 und 38 dem Auge 40 das keimfreie flüssige Medium 17'' in Form einer geeigneten Salzlösung zugeführt werden (Fig.2).

An dieser Stelle wird darauf hingewiesen, dass die Funktionselemente der Irrigationseinheit 45 sowie die Funktionselemente der Aspirationseinheit 75 jeweils auf einem entsprechend als Kassette ausgebildeten und in das Gehäuse 1 (Fig.1) einschiebbaren Trägerelement angeordnet sind. In eingeschobenem Zustand des einzelnen Trägerelements (nicht dargestellt) sind die Leitungen 52,52' der Aspirationseinheit 75 mit der ersten Vakuumpumpe 50 und die Leitungen 41,41' der Irrigationseinheit 45 mit dem Magnetventil 42 verbunden.

Die Funktionsweise der vorstehend beschriebenen Irrigations- und Aspirationseinrichtung 100 wird nachstehend beschrieben:

In einer ersten Phase wird ein Luftpolster, welches sich in einem die Leitungen 48' und 51, 51' sowie den ersten Filter 55 umfassenden System der ersten Pumpeneinheit 80 aufgebaut hat, durch Vakuumieren entfernt. Hierbei werden beide Pumpen 50 und 70 mit nicht dargestellten Mitteln gleichzeitig aktiviert, wobei die zweite Pumpe 70 dabei ausschliesslich das Luftvolumen aus der ersten Pumpeneinheit 80 entfernt. Mittels der ersten Pumpe 50 wird die mit den Gewebeteilchen etc. vermischte Flüssigkeit aus dem Auge 40 entfernt und dem Behälter 54 (Sammelbehälter) zuführt.

Bei einem ersten Arbeitsmodus, bei welchem die Irrigations- und Aspirationseinrichtung 100 mit einer Solldruck-Differenz in der Grössenordnung von 100 bis 600 mmHg (Quecksilbersäule) arbeitet, wird mit vorgegebenem Vakuum-Sollwert das über die Leitung 38 dem Auge 40 zugeführte flüssige Medium zusammen mit den während des operativen Eingriffs anfallenden Gewebeteilchen von der peristaltisch arbeitenden ersten Vakuumpumpe 50, unter Beibehaltung der erforderlichen Feinfühligkeit derselben, über die Leitungen 48,48' und 51,52 abgesaugt und wie bereits vorstehend erwähnt über die Leitungen 52' und 53 dem Behälter 54 zugeführt.

Sobald während des operativen Eingriffs eine Erhöhung des Vakuum-Sollwertes erforderlich wird, beispielsweise von einem Minimum auf ein Maximum des entsprechenden Setwertes, kann zusätzlich zu der bereits laufenden und aktivierten ersten Vakuumpumpe 50 die zweite Vakuumpumpe 70 zugeschaltet und aktiviert werden. Die Zuschaltung der zweiten Vakuumpumpe 70 erfolgt beispielsweise jedoch erst dann, wenn durch die Betätigung eines mit der Vorrichtung 150 in Wirkverbindung stehenden Fusspedals (nicht dargestellt) oder dergleichen eine Soll-Druckdifferenz überschritten wird. Mittels der zugeschalteten zweiten Vakuumpumpe 70 wird die noch vorhandene Luft aus dem gesamten System der ersten Pumpeneinheit 80 abgesaugt und dadurch relativ schnell ein wesentlich höheres Vakuum erreicht. Die Vakuumpumpe 70 der zweiten Pumpeneinheit 80' bleibt bei dem vorstehend beschriebenen ersten Arbeitsmodus nach einem Reflux (Belüftung) solange blockiert bis der Chirurg den Vakuum-Sollwert wieder auf ein Minimum zurückgestellt hat. Hierdurch wird verhindert, dass nach dem ersten Arbeitsmodus mit der automatischen Aktivierung der Belüftung (Reflux) durch das dritte Ventil 65 die zweite Vakuumpumpe 70 unmittelbar wieder aktiviert werden kann.

Bei einem zweiten Arbeitsmodus wird bei der Betätigung des nicht dargestellten Fusspedals oder dergleichen durch den Chirurgen die zweite Vakuumpumpe 70 direkt der bereits aktivierten ersten Vakuumpumpe 50 dann zugeschaltet, wenn ein vorgegebener Vakuum-Sollwert von einem in der Grössenordnung von etwa 40 bis 200 mmHg (Quecksilbersäule) überschritten wird. Eine automatische Belüftung (Reflux) entfällt somit bei dem zweiten Arbeitsmodus.

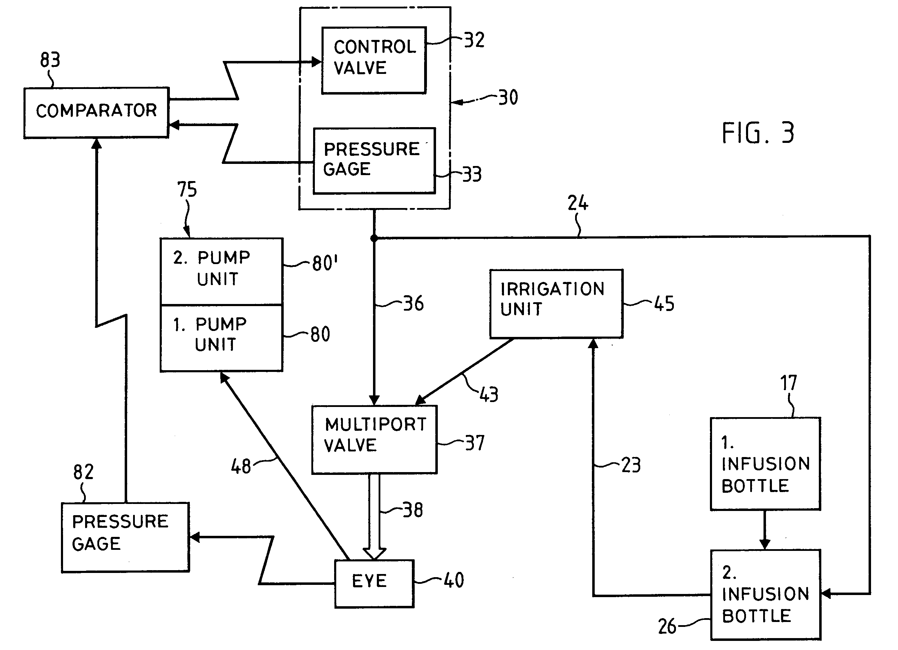

Fig.3 zeigt die als Blockschema dargestellte Irrigations- und Aspirationseinrichtung 100, bei welcher zusätzlich zu den einzelnen in Verbindung mit Fig.2 beschriebenen Funktionseinheiten 10, 30, 45 und 75 ein Druckmessgerät 82 (PRESSURE GAGE) sowie ein damit in Verbindung stehendes Mess- und Vergleichsgerät 83 (COMPARATOR) vorgesehen ist. Mit dem Druckmessgerät 82 wird der momentane intraoculare Druck des Auges 40 ständig überwacht und beispielsweise an dem entsprechenden Anzeige- und Bedienungsfeld 2' des Bildfeldes 2 (display panal) des Gehäuses 1 (Fig.1) angezeigt. Der momentane intraoculare Druck wird zudem erfasst und in dem Mess- und Vergleichsgerät 83 mit dem eingegebenen bzw. mit dem zuletzt gemessenen Wert verglichen. Die ermittelten Werte können beispielsweise zur Änderung einer Eingangsgrösse sowie als umgewandelte elektrische Signale zur Regulierung des mit der Druckquelle 31 in Verbindung stehenden Regel- und Kontrollventils 32 (CONTROL VALVE) herangezogen werden.

Bei der in Fig.1 dargestellten Vorrichtung 150 sind die einzelnen Funktionseinheiten 3, 4, 30 und 45,75 beziehungsweise 85 in nicht näher dargestellter Weise und wie bereits vorstehend erwähnt als Kassette ausgebildet und auswechselbar in das Gehäuse 1 der Vorrichtung 150 einschiebbar. Es besteht jedoch auch die Möglichkeit, dass die einzelnen Funktioneinheiten jeweils als eine eigenständige Baueinheit ausgebildet werden können.

An dieser Stelle wird weiterhin darauf hingewiesen, dass die vorstehende Erfindung anhand eines bevorzugten Ausführungsbeispiels beschrieben wurde und nicht darauf beschränkt ist. Von einem Fachmann durchgeführte Abänderungen und Verbesserungen liegen somit ebenfalls im Rahmen dieser Erfindung.

## Patentansprüche

1. Ophthalmologische Aspirations- und Irrigationseinrichtung für eine Vorrichtung zur Durchführung mikrochirurgischer Eingriffe am Auge eines Lebewesens, wobei die Aspirations- und Irrigationseinrichtung eine Infusionseinheit (10), eine Druckeinheit (30), eine Irrigationseinheit (45) sowie eine mit mindestens einem ersten Filter (55) und mit mindestens einer Vakuumpumpe (50) versehene Aspirationseinheit (75) umfasst, mittels welcher ein dem Auge (40) über eine damit in Verbindung stehende Irrigationsleitung zugeführtes flüssiges oder gasförmiges Medium sowie beim operativen Eingriff anfallende Gewebeteilchen oder dergleichen über mindestens eine mit dem Auge in Verbindung stehende Aspirationsleitung absaugbar sind, **dadurch gekennzeichnet**, dass eine mit dem Auge (40) in Verbindung stehende und mindestens die erste Vakuumpumpe (50), den ersten Filter (55) sowie erste Hauptleitungen (51,51';62,62';63,63';64) umfassende erste Pumpeneinheit (80) sowie eine über zweite Hauptleitungen (67,67') damit in Verbindung stehende zweite Pumpeneinheit (80') vorgesehen sind, wobei die zweite Pumpeneinheit (80') mindestens eine zweite Vakuumpumpe (70) aufweist, die der ersten Vakuumpumpe (50) nachgeschaltet ist.

2. Ophthalmologische Aspirations- und Irrigationseinrichtung nach Anspruch 1, **dadurch gekennzeichnet**, dass in einer die erste Pumpeneinheit (80) mit dem zu behandelnden Auge (40) verbindenden Saugleitung (48,48') ein Ventil (47) zwischengeschaltet ist, welches mit Mitteln zum Unterbrechen und wieder Freigeben des von der Pumpeneinheit (80) bewirkten Saugvorgangs versehen ist.

3. Ophthalmologische Aspirations- und Irrigationseinrichtung nach Anspruch 2, **dadurch gekennzeichnet**, dass das Ventil (47) mit einem die Saugleitung (48,48') unterbrechenden und wieder freigebenden Unterbrecherorgang versehen ist.

4. Ophthalmologische Aspirations- und Irrigationseinrichtung nach Anspruch 1, **dadurch gekennzeichnet**, dass die mit der ersten Vakuumpumpe (50) und dem ersten Filter (55) versehene erste Pumpeneinheit (80) weiterhin einen dem ersten Filter (55) nachgeschalteten zweiten Filter (57) zum Ausscheiden einer Restflüssigkeit, ein erstes Ventil (58) sowie ein Druckmessgerät (59) umfasst.

5. Ophthalmologische Aspirations- und Irrigationseinrichtung nach Anspruch 1, **dadurch gekennzeichnet**, dass zum raschen Belüften des Aspirationssystems der ersten Pumpeneinheit (80) an die eine Hauptleitung (64) eine weitere Leitung (66,66') mit einem zweiten Ventil (60) und einem dritten Ventil (65) angeschlossen ist.

6. Ophthalmologische Aspirations- und Irrigationseinrichtung nach Anspruch 5, **dadurch gekennzeichnet**, dass dem zweiten Ventil (60) für eine gedrosselte Belüftung des Aspirationssystems der ersten Pumpeneinheit (80) ein Drosselorgan (61) vorgeschaltet ist.

7. Ophthalmologische Aspirations- und Irrigationseinrichtung nach Anspruch 5, **dadurch gekennzeichnet**, dass das dritte Ventil (65) mit Mitteln für eine beschleunigte Belüftung des Aspirationssystems der ersten Pumpeneinheit (80) versehen ist.

8. Ophthalmologische Aspirations- und Irrigationseinrichtung nach Anspruch 1, **dadurch gekennzeichnet**, dass die zweite Pumpeneinheit (80') die zweite Vakuumpumpe (70) umfasst und über die Hauptleitung (67) unter Zwischenschaltung eines Drosselorgans (69) und eines Ventils (68) mit der einen Hauptleitung (64) der ersten Pumpeneinheit (80) wirkverbunden ist.

9. Ophthalmologische Aspirations- und Irrigationseinrichtung nach den Ansprüchen 1 bis 8, **dadurch gekennzeichnet**, dass die erste Pumpeneinheit (80) mit den einzelnen miteinander in Verbindung stehenden Funktionselementen (55;57;58;59; 60;61;65) sowie die zweite Pumpeneinheit (80') mit den einzelnen miteinander in Verbindung stehenden Funktionselementen (68;69;70) als eine mit der ersten Vakuumpumpe (50) in Wirkverbindung stehende Baueinheit ausgebildet sind.

10. Ophthalmologische Aspirations- und Irrigationseinrichtung nach Anspruch 1, **dadurch gekennzeichnet**, dass zum Erfassen des momentanen Augendrucks ein Druckmessgerät (82) vorgesehen ist, welches mit einem der Druckeinheit (30) vorgeschalteten und damit in Wirkverbindung stehenden Mess- und Vergleichsgerät (83) verbunden ist.

11. Verfahren zum Betreiben der ophthalmologischen Aspirations- und Irrigationseinrichtung nach den Ansprüchen 1 bis 10, **dadurch gekennzeichnet**, dass in einer ersten Phase und bei gleichzeitiger Aspiration der Flüssigkeit aus dem Auge (40) ein im System der ersten Pumpeneinheit (80) vorhandenes Luftpolster von einer nachgeschalteten zweiten Pumpeneinheit (80') abgesaugt und entfernt, mindestens aber teilweise abgesaugt und entfernt wird.

12. Verfahren zum Betreiben der ophthalmologischen Aspirations- und Irrigationseinrichtung nach Anspruch 11, **dadurch gekennzeichnet**, dass die zweite Pumpeneinheit (80') in Abhängigkeit eines vorgegebenen Solldruck-Wertes, vorzugsweise erst beim Überschreiten desselben der bereits aktivierten ersten Pumpeneinheit (80) zugeschaltet wird.

13. Verfahren zum Betreiben der ophthalmologischen Aspirations- und Irrigationseinrichtung nach Anspruch 12**, dadurch gekennzeichnet**, dass als Solldruck ein in der Grössenordnung von 100 bis 600 mmHg (Quecksilbersäule) liegender Wert vorgegeben wird.

14. Verfahren zum Betreiben der ophthalmologischen Aspirations- und Irrigationseinrichtung nach Anspruch 11, **dadurch gekennzeichnet**, dass die zweite Pumpeneinheit (80') mit einem vorgegebenen Solldruck der bereits aktivierten ersten Pumpeneinheit (80) zugeschaltet wird.

15. Verfahren zum Betreiben der ophthalmologischen Aspirations- und Irrigationseinrichtung nach Anspruch 14, **dadurch gekennzeichnet**, dass für die Zuschaltung des Solldruckes ein in der Grössenordnung von 40 bis 200 mmHg (Quecksilbersäule) liegender Wert vorgegeben wird.

16. Verfahren zum Betreiben der ophthalmologischen Aspirations- und Irrigationseinrichtung nach den Ansprüchen 11 bis 15, **dadurch gekennzeichnet**, dass die erste Pumpeneinheit (80) in Abhängigkeit der einzelnen Verfahrensschritte durch mehrere im System angeordnete Mittel gedrosselt und beschleunigt belüftet wird.

17. Verfahren zum Betreiben der ophthalmologischen Aspirations- und Irrigationseinrichtung nach Anspruch 11, **dadurch gekennzeichnet**, dass die Aspiration der Flüssigkeit aus dem Auge (40) unmittelbar im Bereich desselben durch ein in der Saugleitung (48,48') zwischengeschaltetes Unterbrecherorgan (47) unterbrochen und wieder freigegeben wird.

18. Verfahren zum Betreiben der ophthalmologischen Aspirations- und Irrigationseinrichtung nach Anspruch 11, **dadurch gekennzeichnet**, dass der momentane intraokulare Druck des Auges (40) erfasst und mit dem vorgegebenen oder mit dem zuletzt gemessenen Wert des Druckes verglichen und dieser zur Regulierung oder zur Korrektur einer vorgegebenen Eingangsgrösse herangezogen wird.
